# EUROPEAN PATENT APPLICATION

(11) **EP 1 408 436 A1**
(43) Date of publication of application: **14.04.2004**
(21) Application number: 02741419.2
(22) Date of filing: 05.07.2002
(51) Int. Cl.: G06F 17/60, A61G 12/00, A61B 5/00

(54) **PATIENT INFORMATION MANAGEMENT APPARATUS AND METHOD**

(30) Priority: 06.07.2001 JP 2001205818
(71) Applicant: Koninklijke Philips Electronics N.V., 5621 BA Eindhoven (NL)
(72) Inventor: MIYASAKA, Katsuyuki, Tokyo 154-0004 (JP); SUGANUMA, Kunio, c/o Sota Systems Kabushiki Kaisha, Shinjuku-ku, Tokyo 160-0023 (JP); KOMIYA, M., c/o Philips Medical Systems K. K., Tokyo 108-8507 (JP)
(74) Representative: Volmer, Georg, Dipl.-Ing.
(86) International application number: PCT/JP2002/006862
(87) International publication number: WO 2003/005265

(57) **Abstract**

A health care worker is able to manually input data by zooming a graphical representation of vital data, moving a cursor at a predetermined position using a mouse or the like, and clicking the mouse. If it is confirmed that the value can be correct, then a setting button is clicked. Alternatively, it may be canceled. For the cancellation, a click button is clicked. By the way, it can be also observed as an output on a monitor (Steps from S11 to S16). In this case, the fact of being confirmed is displayed over the screen on which the monitor output of the vital data is displayed.

## Description

### Technical Field

This invention relates to a system for managing patient information which receives, for storage, highly diversified, highly dense information of patients fed automatically or manually via terminals from the wards of a hospital where seriously sick patients are cared, particularly from ICU or NICU; and displays the data on a mode appropriately chosen according to a given purpose, thereby helping medicare personnel to construct a therapeutic plan or care plan, or allowing them to utilize the data as a material for their electronic medical records.

### Background Art

The known system for managing patient information includes, for example, a system developed by Dr. Tait of Hospital for Sick Children, Toronto, Canada. This patient information management system was developed around 1980, and based on a client-server network constituted of Macintosh® computers actually used in the ICU of this pediatric hospital until around 1998.

According to this information management system, a physician can instruct a nurse a method how to conduct the bolus or dripping injection of a medicine to a patient. However, although the instruction can convey sufficient information about the mode of injection such as single injection, repeated injection, etc., its information about the time schedule of injection is not always satisfactory.

According to this system, a default display mode is predetermined for each clinical category. Therefore, the user cannot exchange the display mode of a patient classified to a certain category for the display mode of a different category. Moreover, according to this known system, a physician cannot inform a nurse of what precautions should be taken, for a given medicine, as regards the risk of its causing an adverse interaction when it is applied to a patient suffering from a particular disease or receiving a particular medicine.

Furthermore, according to this system, a physician cannot inform a nurse of what precautions should be taken, for a given medicine, as regards the risk of its invoking an adverse response when it is applied to a patient through a certain route (a medicine may be safely applied to a patient, when injected through a central vein but may evoke an adverse response when injected through a peripheral vein, or vice versa, or a medicine may be safely applied to a patient when injected alone through a vein, but may cause an adverse interaction when injected through the same vein within a certain period after another medicine has been injected through the same vein, depending of the kind of the latter medicine). Still further, according to this system, the user cannot choose desired display modes from among various display modes available, to combine them.

Another known patient information management system includes one provided by Fujitsu (EG-Main EX). This system spreads widely among university hospitals and big private hospitals having a capacity sufficiently large to accept 200 or more beds. According to this system, the user or physician can instruct nurses the bolus or dripping injection of medicines, and prescriptions.

If a studied patient is so seriously sick that his/her condition changes from one moment to another, it is necessary for treating the patient properly to follow the pathological condition of the patient moment by moment. For this purpose, the physician must monitor at regular intervals the vital condition of the patient, and manage the thus obtained data in such a manner as to allow him/her to timely take a proper action for any pathological change.

However, according to said patient information management system, the physician cannot always take proper action timely for a sudden pathological change particularly of a seriously sick patient.

In addition, in a ward such as PICU where seriously sick patients are cared, even if such a patient information system as described above is introduced, the user or physician must deal with two different kinds of data, that is, data provided through a hospital information system (HIS) and data provided through a department information system (DIS), and the user must determine, for a given data, to which category it should be classified. This may disturb the concentration of the user or physician, which should be exclusively directed towards his/her patients. It is true that introduction of a computer system such as HIS and/or DIS in a hospital ensures efficient management of clinical works as well as clerical works of the hospital. However, it has the risk of causing adverse side-effects: an operator may feed an erroneous input, or miss an input, or a physician may give an erroneous instruction or miss an instruction, which may lead to a serious blunder. Even if the system is so configured that clinical data are automatically fed to the system, the system will cause another frustration: data fed from various sources become so voluminous and complicated that the physician might not be able to rightly grasp the pathological condition of his/her patients.

### Disclosure of Invention

In view of the above problems encountered with the conventional systems, this invention had been proposed. The present inventors aimed at providing a patient information management system, which is "directly helpful for the promotion of the patient's safety and health." They analyzed the procedures a medical professional will take when he/she must treat a patient in ICU, and developed, based on the analysis results, a system which can minimize the risk of making medical blunders as a result of erroneous input/instructions, missed input/instructions or duplicate input/instructions. One object of this invention is provided such a patient information system.

Another object of this invention is to provide a "versatile system widely applicable to various medical departments": in the pediatric department physicians must deliver a wide variety of instructions regarding injections and therapies, and orders of materials and facilities incessantly, and clerks and nurses must check those instructions and orders, and practice the instructions and dispatch the orders to external providers, and staff of ICU must command a number of widely different therapeutic courses.

Yet another object of this invention is to provide an information system smoothening the entire works of a hospital capable of delivering security-insured, electronic medical records, safely storing the medical data, and providing a standard interface through which it can communicate with any external systems. This system will ensure not only the improved efficiency of clerical works of the hospital, but also the safe management of patients including pediatric patients.

The above objects of this invention can be achieved by providing an apparatus for managing patient information comprising at least one condition detecting means for detecting patient conditions; detected value displaying means for outputting a detected value obtained in said condition detecting means to display the detected value on a display screen; corrected value inputting means for inputting a corrected value of the detected value in the display screen displayed by said detected value displaying means corresponding to said condition detecting means; and corrected value displaying means for displaying said corrected value inputted by said corrected value inputting means on said display screen that said detected value is displayed.

The condition detecting means for detecting patient condition comprises applying, on various appropriately chosen spots on the body of a patient, detection means or sensors, e.g., of an electrocardiography meter or of manometer adapted for detecting specified signals peculiar to the spots, and delivering the received signals to a bedside monitor for inspection. This means enables the detection of blood pressures at specified spots or of the heartbeat, and the delivery of the detected signals as output. The monitor receives signals from those detection means, and delivers the signals to other external devices.

It is empirically known that an output value displayed on the monitor does not always reflect rightly the condition of a patient. It is of course prohibited to artificially modify the output value on the monitor, because it constitutes a fake. If a medical practitioner determines that a given output value on the monitor does not reflect the condition of his/her patient, based on his/her own experience or on the results of other clinical tests, and has in mind a certain possibly right value, he/she will want to store his/her expected right value together with the actual value on the monitor for later reference. To meet such a demand, the system of the present invention allows the user to feed his/her expected right value in addition to the actual output on the monitor. The system of the present invention further allows the input fed by the user to be displayed on the monitor together with the actual output, which will be a great help for the user in constructing a therapeutic plan.

The situation where the medical practitioner suspects an output on the monitor does not rightly reflect the true condition of his/her patient includes the following: the monitor itself is disordered, or the output on the monitor is at a transitional phase prior to recovery to a normal, constant phase. Besides the above situations, it is empirically known that the output on the monitor becomes unstable for a certain period after treatment. During that phase, the patient him/herself also becomes so unstable that it is often difficult to determine the true condition of the patient solely dependent on an output on the monitor. To meet such a situation, the physician asks the patient about his/her condition, or palpitates his/her pulses to check or determine the true condition of the patient. According to the system of the present invention, the user can feed his/her expected right condition of the patient obtained based on his/her own examination results. However, since this value is not the direct output on the monitor, the system of the present invention allows the input fed by the user to be displayed in parallel with the actual output on the monitor.

The corrected value inputting means allows a corrected value fed by the user to be displayed in parallel with a detected value provided by the condition detecting means. Then, the user or medical practitioner can compare the corrected value fed by him/herself with the actually detected value on the monitor. If the difference between the two values is too large to be ignored, the user or medical practitioner has his/her attention aroused towards the cause underlying the difference. This urges the user or medical practitioner to be prudent in the treatment of the patient, which will insure the safe and proper treatment of the patient. If the user finds no discrepancy between an output on the monitor and his pathological image of the patient, this means that the user finds the system reliable, and the treatment based on the reliable system will be also reliable.

A preferred embodiment may further comprise a display stopping means which can stop the display of detected values of chosen parameters. The operator may activate the display stopping means in the following situation. If a patient receives a medicine, his/her physical condition changes temporarily. Then, parameters representing the functional aspects of his/her body may also change. Then, the output on the monitor will not rightly reflect the true condition of the patient. If the user took this output as reflecting the true condition of the patient and constructed a therapy based on such data, that therapy would result in a failure. It is more right for the user to activate the display stopping means and to wait for a certain period until the physical condition of the patient returns to a normal, constant state, and parameters representing the functional aspects of the patient's body become stable. Indeed, activation of the display stopping means may prevent the occurrence of a blunder which might occur if the output on the monitor is relied on heedlessly. In addition, the system allows the user to activate the display stopping means for a certain predetermined period, and to freely alter the period. Once the user feeds the input specifying when and how long the display stopping means is activated, the system automatically activates the display stopping means at each specified time for a specified period. However, it is also possible for the user to manually activate and inactivate the display stopping means.

The condition detecting means preferably includes at least two selected from the group consisting of heartbeat detecting means for detecting the heartbeat of a patient; arterial pressure determining means for determining the arterial pressure of a patient; oxygen-saturation-in-blood determining means for determining the oxygen saturation in blood of a patient; central venous pressure determining means for determining the central venous pressure of a patient; left atrium pressure determining means for determining the left atrium pressure of a patient; pulmonary artery pressure determining means for determining the pulmonary artery pressure of a patient; intracranial pressure determining means for determining the intracranial pressure of a patient; and respiratory rate measuring means for measuring the respiratory rate of a patient. These parameters serve as the vital sign of a patient, and represent the basic functions of the patient's body. If the sequential changes of those parameters are maintained on display, it will be possible for the user to closely follow the pathological changes of the patient and thus to timely apply proper treatment to the patient.

As another feature, the present invention provides an apparatus for managing patient information comprising treatment schedule displaying means for displaying a treatment schedule as a function of time; performed treatment displaying means for displaying actually performed treatment as a function of time; and comparing means for comparing the actually performed treatment with the treatment schedule, checking whether the two are in agreement, and displaying the comparison result.

The above apparatus has a first display screen for displaying the treatment schedule, actually performed treatment and comparison result, and a second display screen for displaying the spots on a human body to which treatment should be applied, and the first and second display screens are disposed side by side to be viewed simultaneously by an observer.

With this display system, the observer can see mutually interrelated, but qualitatively different data presented on different screens at the same time, and thus rightly grasp what treatment has been performed on a patient, and can construct a proper therapeutic plan based on this right understanding, which may lead to the prevention of accidental medical blunders, and contribute to the establishment of highly reliable medicine.

Preferably, the above apparatus further comprises a display linking means for linking the treatment schedule, actually performed treatment and comparison result on the first display screen with the spots on a human body on the second display screen. This apparatus makes it easier for the user to check the history of treatment hitherto performed on a patient.

Preferably, with the above apparatus, the treatment schedule displaying means displays the scheduled treatments in a graphic form.

Preferably, with the above apparatus, the performed treatment displaying means displays the actually performed treatments in a graphic form. This apparatus makes it easier for the user to check to what extent the scheduled treatments have been performed.

As yet another feature, the present invention provides an apparatus for managing patient information comprising at least one condition detecting means for detecting patient conditions; detected value displaying means for outputting a detected value obtained in said condition detecting means to display the detected value on a display screen; corrected value inputting means for inputting a corrected value of the detected value in the display screen displayed by said detected value displaying means corresponding to said condition detecting means; corrected value displaying means for displaying said corrected value inputted by said corrected value inputting means on said display screen that said detected value is displayed; treatment schedule displaying means for displaying a treatment schedule as a function of time; performed treatment displaying means for displaying actually performed treatment as a function of time; and comparing means for comparing the actually performed treatment with the treatment schedule, checking whether the two are in agreement, and displaying the comparison result; and display screen switching means for arbitrarily choosing, by switching, one or two or more among the display screen for displaying detected and/or corrected values, display screen for displaying the treatment schedule, display screen for displaying actually performed treatment, and display screen for displaying the comparison result.

With this apparatus, the user can freely choose, by switching, one or two or more among the display screens for displaying detected and/or corrected values, treatment schedule, actually performed treatment and comparison result. Thus, the user can rightly grasp what treatment has been performed on a patient in parallel with the sequential change of the patient's condition, and can construct a proper therapeutic plan based on this information and apply reliable treatment based on the plan.

As yet another feature, the present invention provides a method for managing patient information comprising the steps of detecting at least one patient condition using condition detecting means and a monitor connected to said condition detecting means in order to grasp a patient condition and outputting a detected result of said condition detecting means on a screen; correcting a monitor output on the basis of said monitor output displayed and displaying a corrected value on the monitor screen with said monitor output; displaying a treatment schedule as a function of time; displaying actually performed treatment as a function of time; comparing the actually performed treatment with the treatment schedule, checking whether the two are in agreement, and displaying the comparison result; and arbitrarily choosing, by switching dependent on an instruction from the operator, one or two or more among the display screen for displaying detected and/or corrected values, display screen for displaying the treatment schedule, display screen for displaying actually performed treatment, and display screen for displaying the comparison result.

This invention is generally achieved by a general-purpose computer system, and the above functions can be achieved by instructions from the computer, that is, programs properly provided by the computer. Thus, as yet another feature, this invention provides the programs necessary for achieving the above functions.

### Brief Description of the Drawings

Fig. 1 is a sketchy diagram of a patient information management system of the present information.
Fig. 2 is a diagram for outlining the constitutive elements of the patient information management system as shown in Fig. 1.
Fig. 3 shows the organization of display screens of the patient information management system of the present invention.
Fig. 4 shows a basic display screen used in the patient information management system of the present invention.
Fig. 5 is a flowchart of functions the user can effect through the basic display shown in Fig. 4.
Fig. 6 illustrates an example of display to be used in the patient information management system of this invention.
Fig. 7 illustrates another example of display to be used in the patient information management system of this invention.
Fig. 8 illustrates yet another example of display to be used in the patient information management system of this invention.
Fig. 9 illustrates yet another example of display to be used in the patient information management system of this invention.
Fig. 10 illustrates yet another example of display to be used in the patient information management system of this invention.
Fig. 11 lists data elements required for constructing the display of Fig. 10.
Fig. 12 shows a table in a master file of Fig. 11.
Fig. 13 shows an example of a display used in the patient information management system of this invention.
Fig. 14 is a flowchart of functions the user can effect through the display shown in Fig. 10.
Fig. 15 is an example of the flow of information through the patient information management system of this invention.

### Best Mode for Carrying out the Invention

Preferred embodiments of the present invention will be described with reference to attached figures.

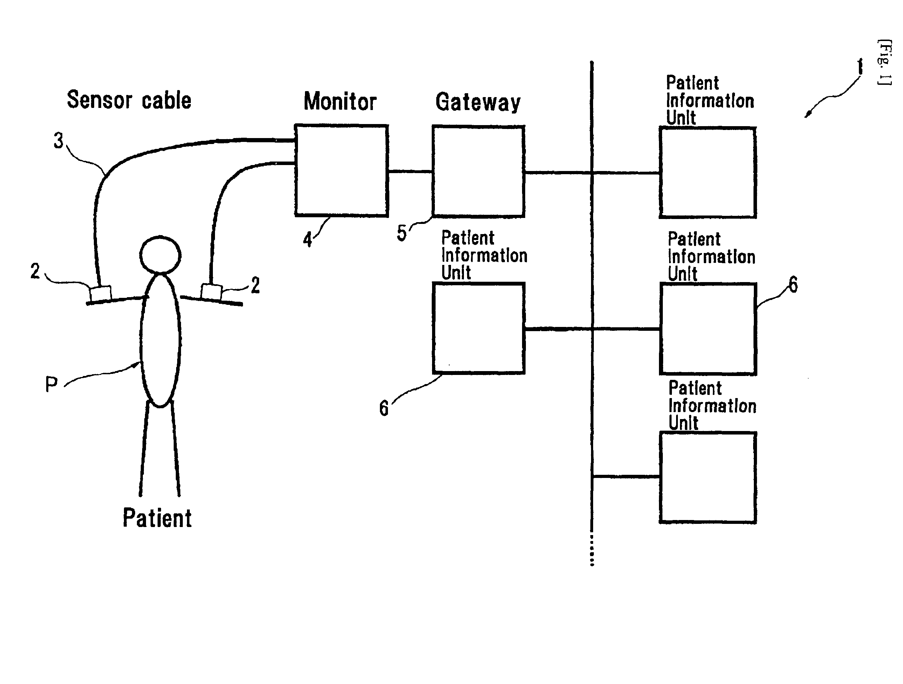

Fig. 1 is a block diagram for outlining a patient information management system 1 (information management system) of the present information. Various sensors 2 are applied on the body of a patient P for monitoring the condition of the patient. Each sensor 2 is connected via a sensor cable to a monitor 4. The monitor 4 receives signals from the sensors 2, and delivers them as patient information to the system. Each output cable from the monitor 4 is connected via a gateway 5 to a network system comprising plural patient information management units 6 (information management unit). The information management system 1 is based on a network of the information units 6. Patient information transmitted from the above sensors is received and managed by a information management unit 6. Usually, one of the information management units 6 serves as a server, and the rest as clients, and overall a server-client system is formed.

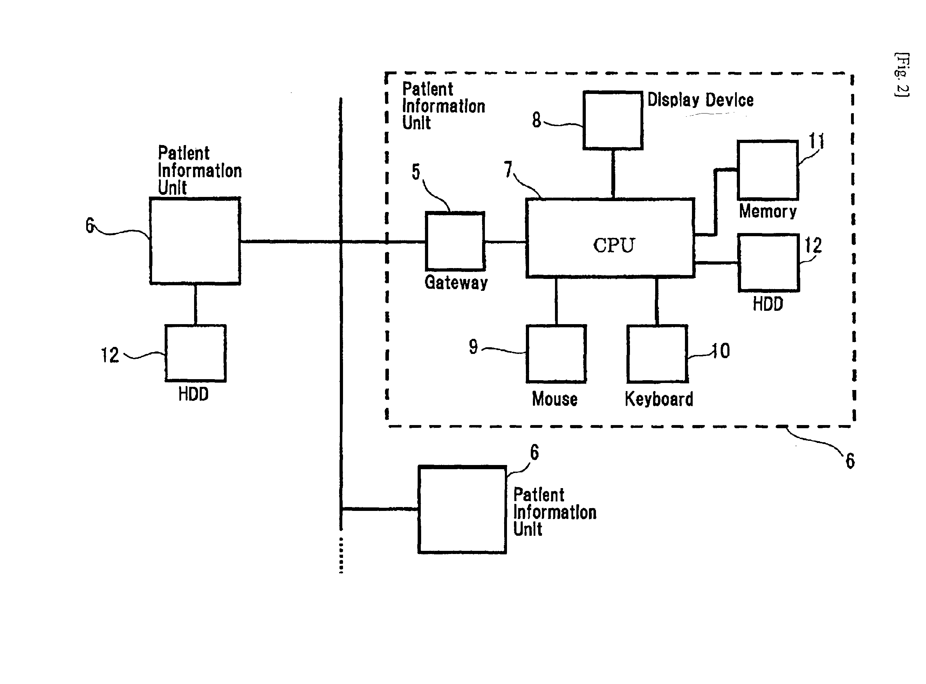

Fig. 2 is a diagram for outlining the constitutive elements of an information management unit 6. Each information management unit 6 consists of a general-purpose personal computer. Namely, each information management unit 6 comprises a CPU 7, a display device 8 connected to the CPU, a gateway 5 for communicating information through networks, a mouse 9, a keyboard 10, a memory device 11 such as a hard disk, and a hard disk drive (HDD) 12.

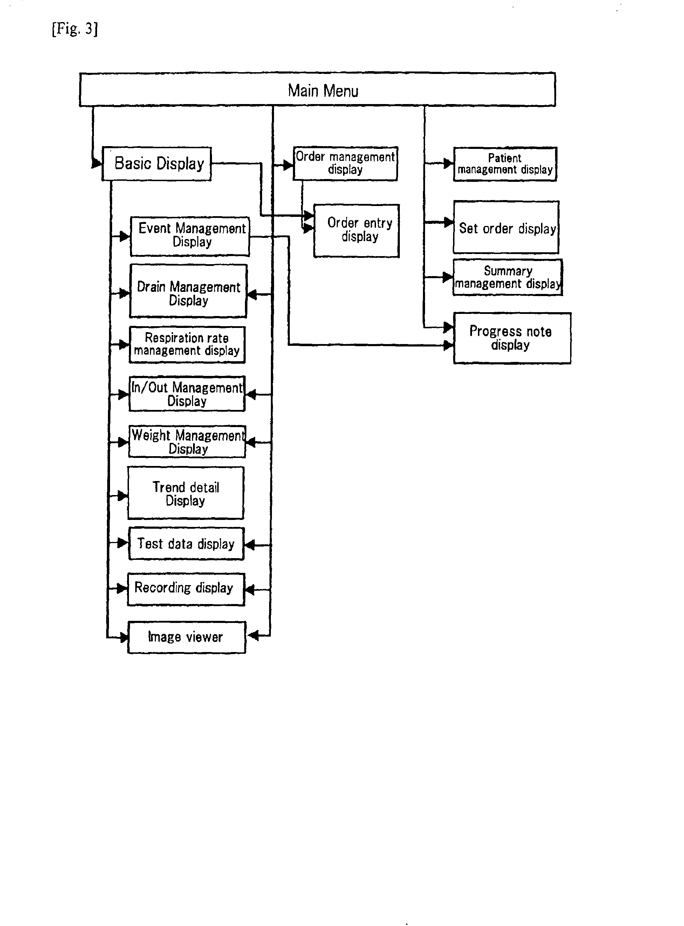

Fig. 3 shows the organization of display screens of an embodiment of the present invention. According to this embodiment, when a personal computer representing the patient information management unit is switched on, a basic display is presented which provides a main menu through which the user can access to any displays available. The user or medical practitioner points to a desired display on the main menu by using an appropriate input feeding device such as a mouse, depending on the medical activity he/she is currently interested in.

If the user so desires, he/she can feed input such as measurement data he/she directly obtained from his/her patient by gaining access to a display specially prepared for the purpose, and feeding necessary input using a mouse.

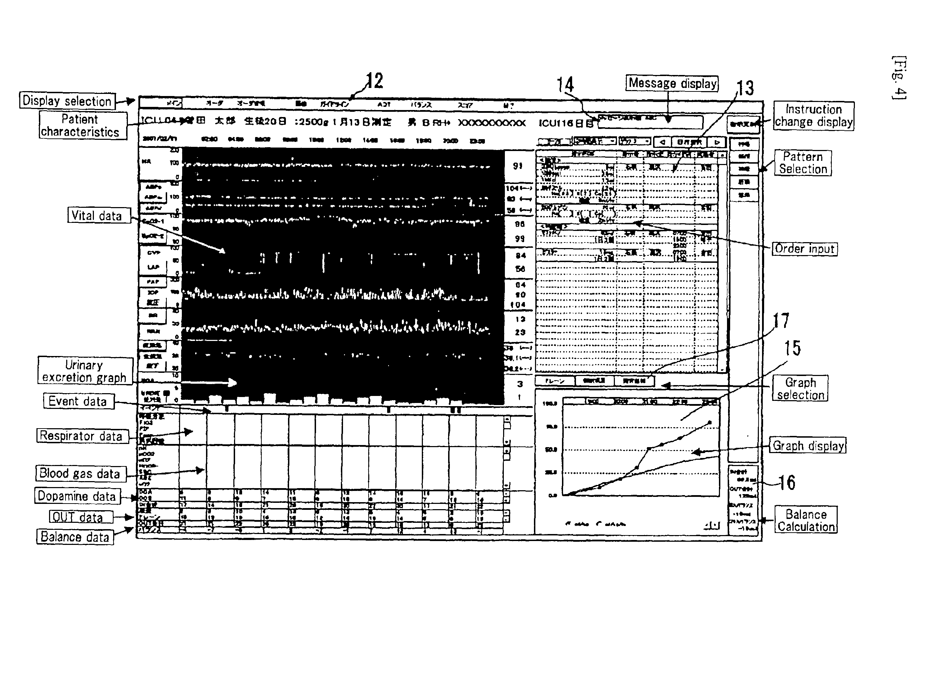

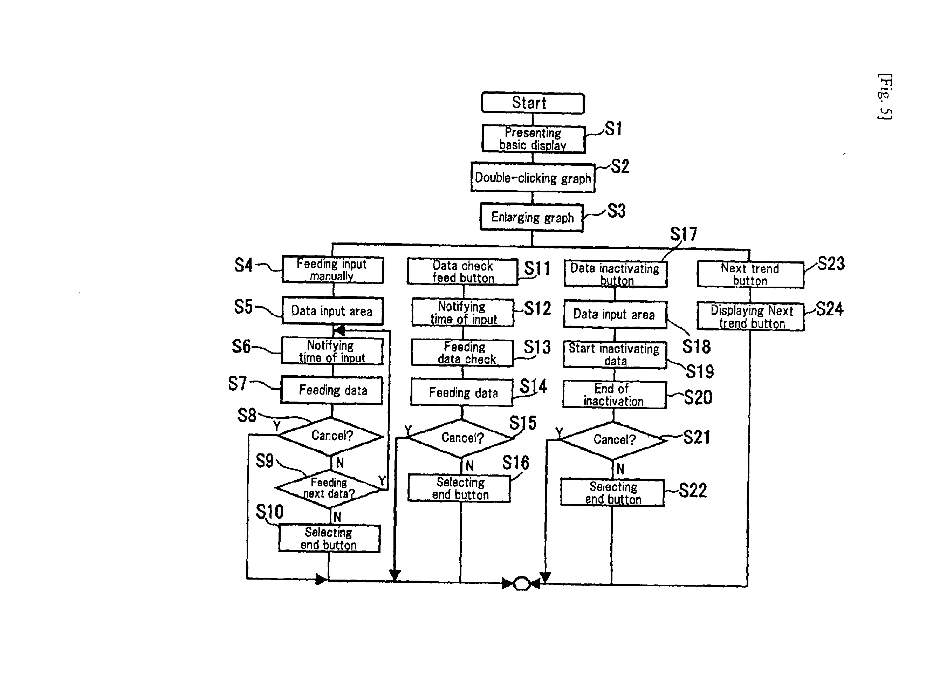

Fig. 4 shows a basic display screen including the main menu. Fig. 5 is a flowchart of functions the user can effect via the basic display shown in Fig. 4.

The manner how the user can operate the unit via the basic display will be described below with reference to Figs. 4 and 5.

This basic display has four features.
1) It shows the sequential changes of the patient condition by presenting the trace records of patient's vital functions transmitted at regular intervals from the sensors applied on the patient's body.
2) It shows a series of clinical data on the same time axis as used for the above trace records. The clinical data includes injection of medicines, urine volumes, results of hematological tests, etc.
3) It shows instruction from physicians regarding dripping injection of a medicine, etc.
   The user can check the instructions from physicians by comparing them with the data given in 1) and 2).
4) It shows various patient data in graphs.

The main menu 12 appears on the top bar of this basic display of Fig. 4. The bar just beneath the top bar of Fig. 4 presents the basic characteristics of the patient.

In this particular embodiment, the vital data includes HR (heart rate), ABP (arterial blood pressure), SpO2 (saturated oxygen in blood), CVP (central venous pressure), LAP (left atrial pressure), ICP (intracranial pressure), PAP (pulmonary arterial pressure), RR (respiratory rate), etc. The abscissa represents the time axis. In this particular embodiment, data recorded for last 24 hours can be displayed. The ordinate represents the height of individual trace records. Beneath the trace records there is provided a histogram representing the sequential changes of urine volume. Beneath the histogram there is provided treatment data (event data), which indicates, actually performed treatments arranged on the same time axis. Beneath the event data, there are provided data of an artificial respirator, blood gas, dopamine, excretion, and balance (between food intake and excretion).

In a region right to the vital data display panel, there is provided a display panel for displaying a history of treatment. This panel carries minute data regarding individual treatments actually performed: instructions from physicians, the name of instructing physicians, the name of nurses receiving the instructions, the name of personnel actually putting the instructions into practice, the name of medicines actually applied, the dose of the medicines, etc., as well as the times of medicine application.

Above the display panel 13 for the treatment history, there is provided a window or message displaying area 14 to receive a message from the user. The display panel for the treatment history has different windows to receive data regarding treatment, the site to which treatment was applied, etc. Right to the display panel for the treatment history there is provided a vertical strip comprising buttons through which the user can choose any desired display available to the unit.

Below the display panel 13 for the treatment history, there is provided a display panel 15 which presents the sequential change of a desired parameter in the form of a graph.

In this panel for graphical displaying, the abscissa represents the time axis, and the ordinate represents the height or intensity of a desired parameter which represents a function of the patient's body. In the particular example shown in the figure, the data regarding the drainage of bodily fluid is plotted as a function of time. The user can change the graphical display by depressing a button on the bar 17 just above the display panel here concerned. On the lower right corner of the screen there is provided a display panel 16 for displaying numerical data regarding the nutritional balance of the patient.

The user can obtain the basic display shown in Fig. 4 by clicking an appropriate button on the menu on the top bar, and this corresponds to step S1 in the flowchart of Fig. 5 (step S1).

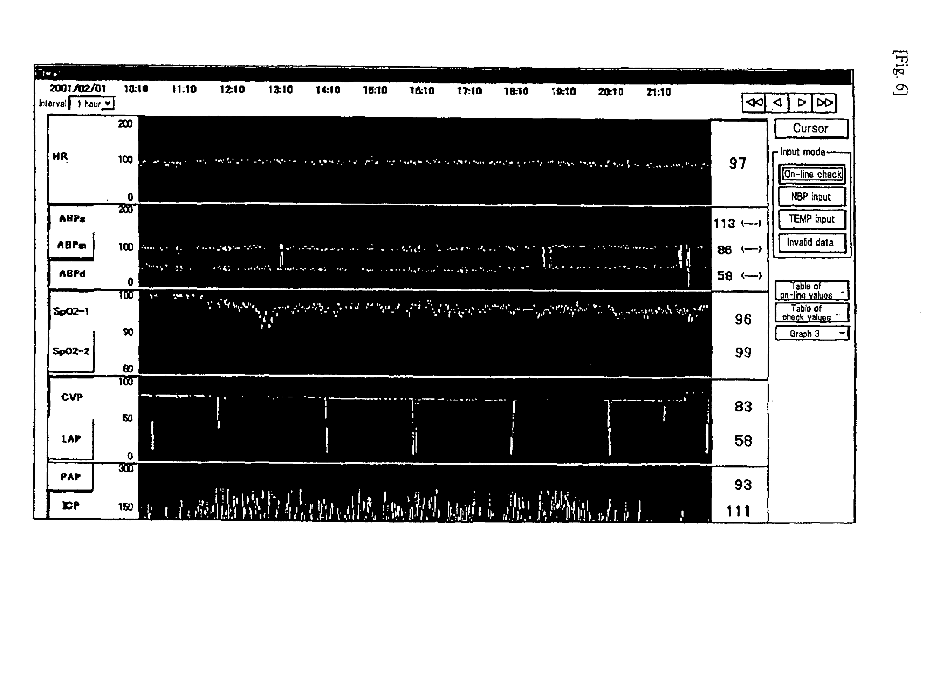

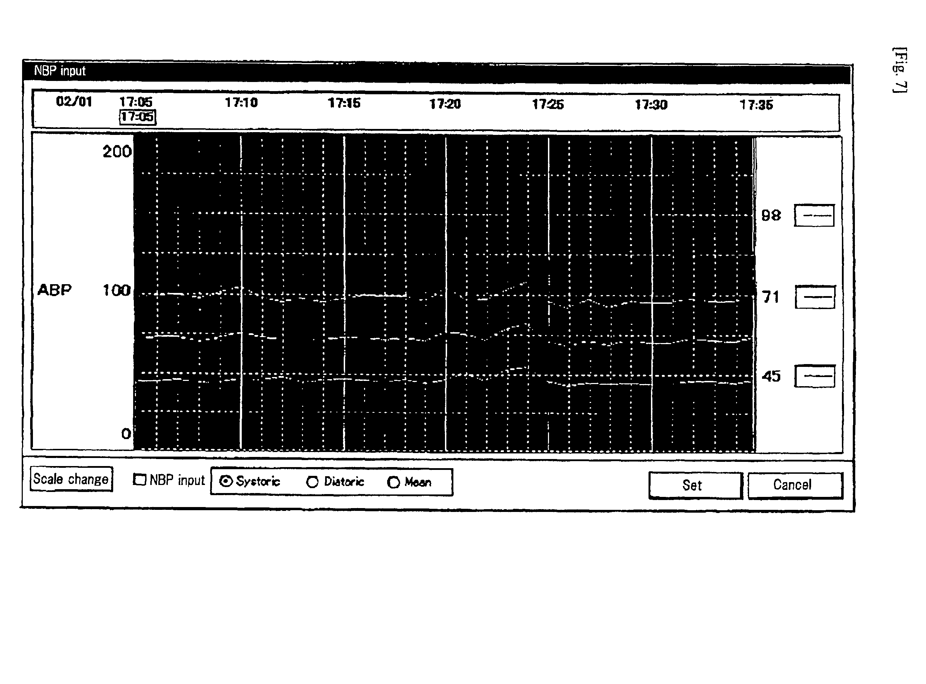

If the user double-clicks while placing a cursor on the display panel for trace records, the display panel for trace records will be enlarged (steps S2 and S3 of Fig. 6). The user can display desired trace records of vital functions on this enlarged display panel (Fig. 7). The user or medical practitioner may feed his/her data he/she obtained on his/her own accord via the display of Fig. 7, and then the data will be displayed on the enlarged display as shown in Fig. 6 or Fig. 7, in parallel with the data transmitted from the sensors.

When the user wants to feed his/her personal data, he/she must take the steps as shown in steps S4 to S10 of the flowchart of Fig. 5. Specifically, the user or medical practitioner enlarges the display panel for the trace records of vital functions, moves the cursor to a desired position on the panel, and clicks the mouse. This operation enables the entry of the user's personal data onto the display panel. Then, the user checks whether the input is rightly fed, and, after confirming that the entry of data has been properly achieved, he/she clicks the confirmation button. Then, the fed data is confirmed. However, the user can modify the confirmed data by clicking the cancel button (see Fig. 7). The user can also check the height or intensity of trace recodes displayed on the monitor (steps S11 to S16). The height or intensity actually checked for a trace record is marked on the same trace record on the display (marked by a round dot in Fig. 6).

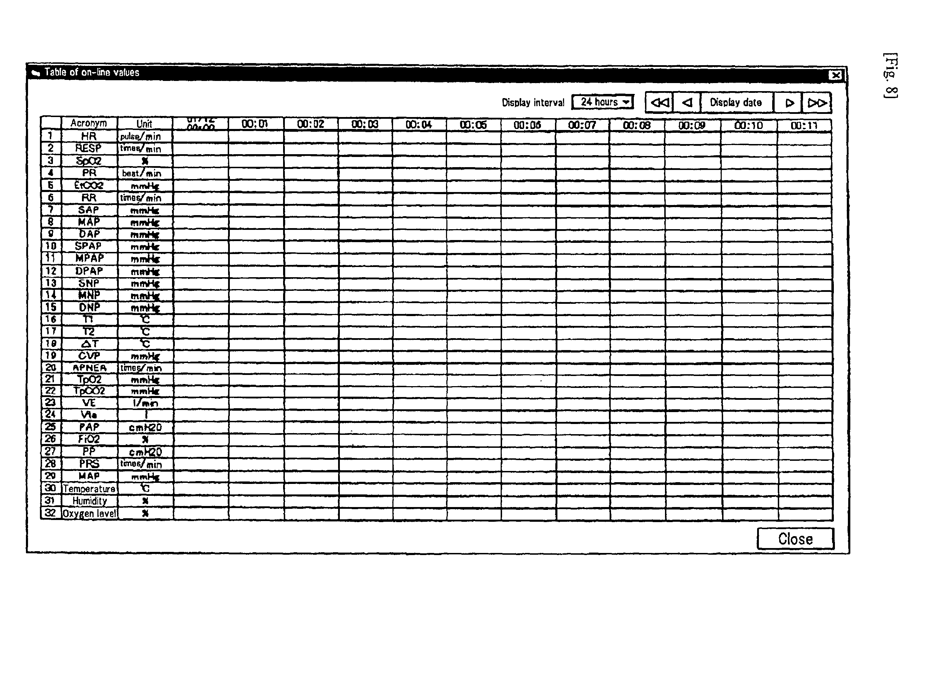

As shown in Fig. 8, the user can extract any point on a trace record continuously transmitted from the sensors applied on the patient's body as a numerical value. For example, it is possible for the user or physician or nurse to actually determine by him/herself the heart rate of the patient, and to compare the result with the numerical data obtained from the heart rate record on display, and to further display the his/her own measurement in parallel with the trace record based on the sensors.

Moreover, if the trace records of vital functions of the patient show levels too abnormal to be taken as reflecting the true condition of the patient, it is possible for the user or physician to instruct the unit to discontinue the recording of vital functions for a certain specified time. Such abnormal surges may appear immediately after the injection of a medicine, or treatment.

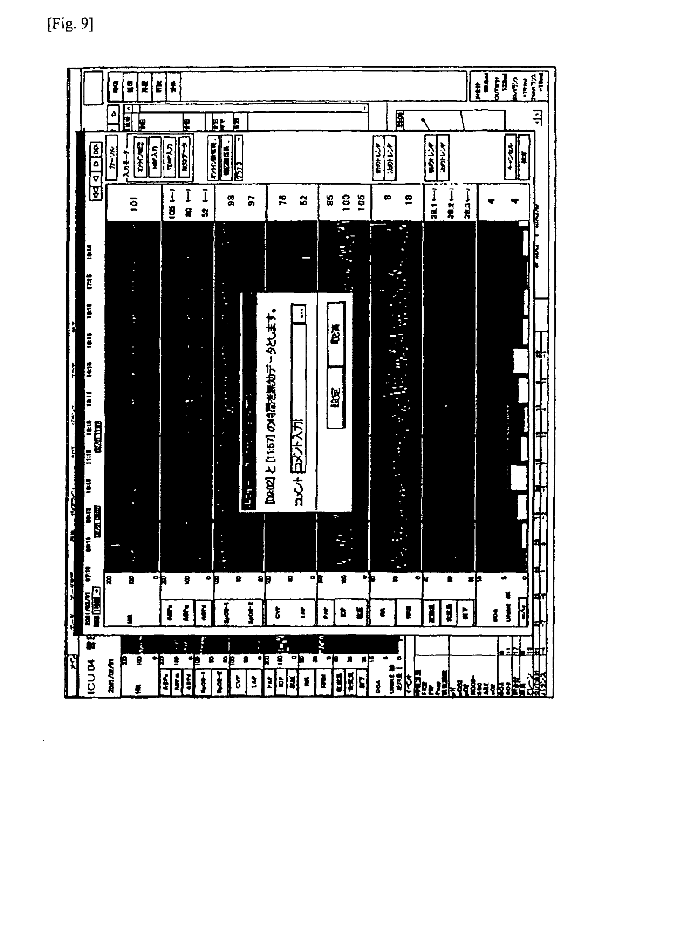

As desired, the user can instruct the unit to discontinue recording by manually feeding the instruction via a display (see Fig. 9 and steps S17 to S22 of Fig. 5). The user can add, to the instruction, the reason why he/she wants discontinuation of recording (see Fig. 9).

If the user wants to exchange a trace record of a vital function for a trace record of another vital function, he/she may click a button which activates the trace record of the vital function he/she wants to see (step S23 and S24).

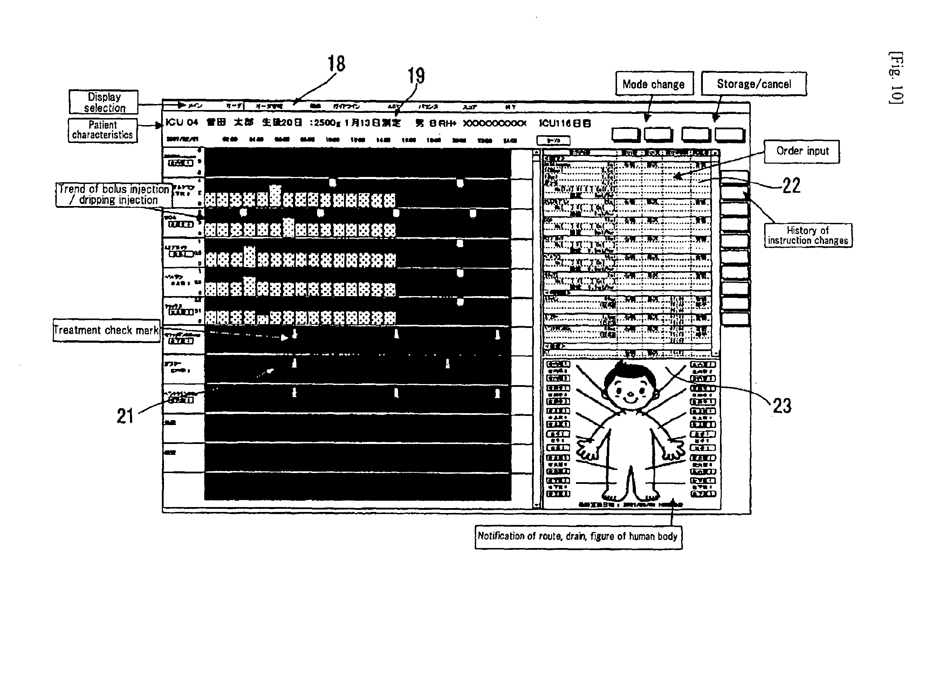

Let's assume that the user transfers to another basic display screen by clicking a different button on the menu bar (Fig. 10). The operations achievable via this basic display screen will be described below.

If the user clicks a button for treatment history on the main menu bar of Fig. 4, he/she will move to another basic display representing treatment history as shown in Fig. 10.

The display panel for treatment history has following functions.
1) It displays instructions from physicians in a sequential order.
2) It displays a diagram of a human body and manages the routes of dripping injection based on the diagram.
3) It shows instructions 1) from physicians in parallel with the data 2).

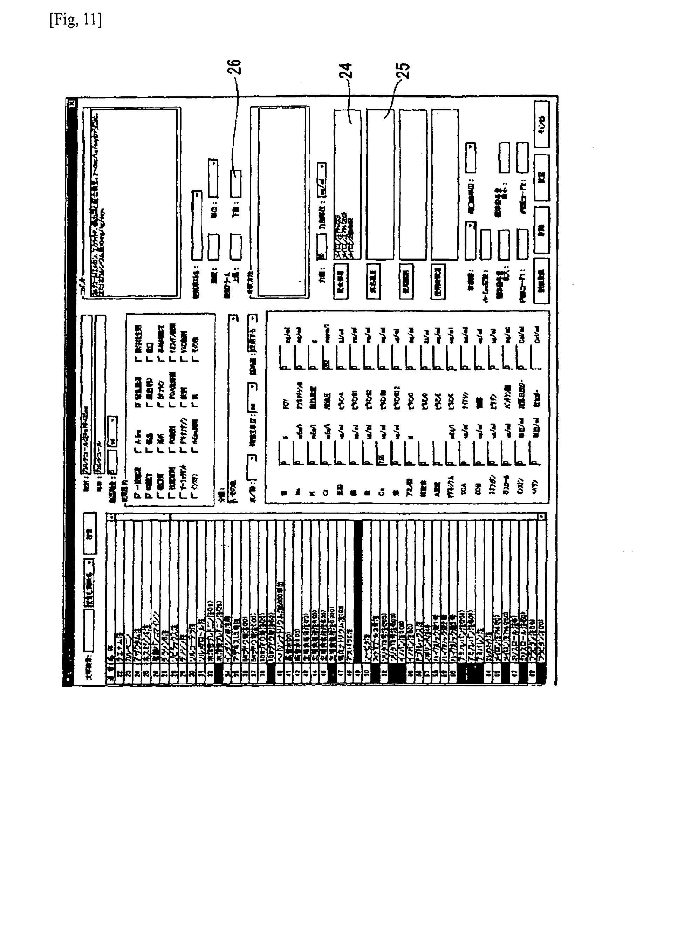

Fig. 11 lists data elements required for constructing the display of Fig. 10. The elements necessary for the preparation of data displayed in Fig. 11 include a control portion 101 for controlling the overall system, a memory portion 102 for storing various data, an input portion 103 for receiving input fed by physicians, exchange timing determining portion 104 which determines the timing of exchange of individual medicine containers, threshold determining portion 105 which determines threshold values for individual medicines to be administered, a display 107 for displaying data as shown in Fig. 10, and a display control portion 106 for controlling the data to be displayed on the display 107.

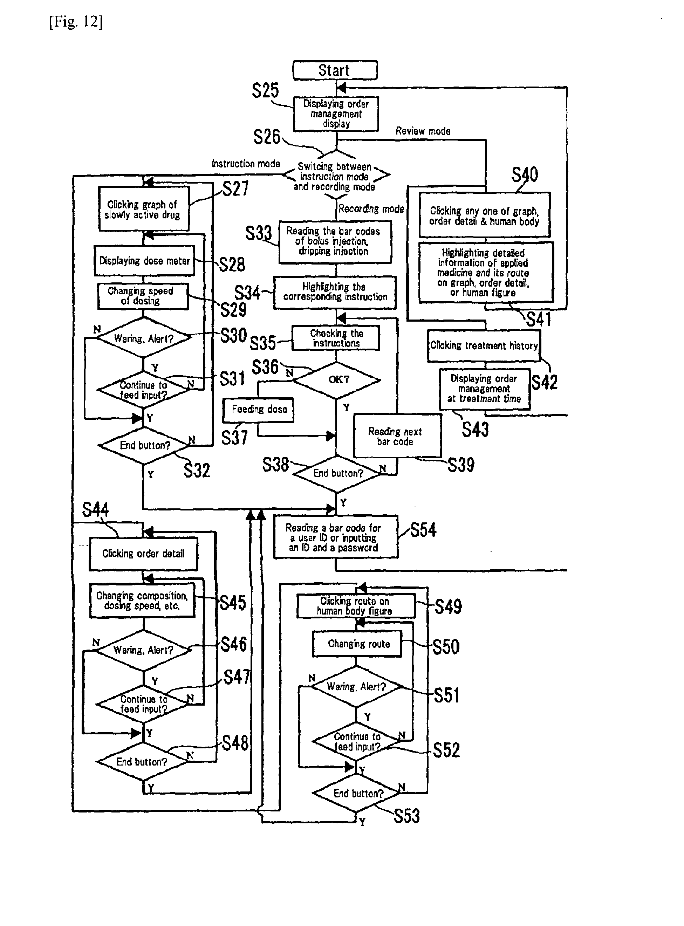

The memory portion 102 comprises a patient database (DB) for storing patients' data, and a master file 1022 linking medicines and their ingredients with their adverse interactions with other medicines or medical conditions. Specifically, the table of the master file 1022 is as shown in Fig. 12.

Let's assume that the user wants to feed the dose of a medicine to be injected via the graphical display panel 21. The user instructs a medicine to be injected to the unit. Then, the controlling unit 101 looks through the master file 1022, and informs the threshold value determining portion 105 of the ingredients of the medicine. The threshold value determining portion determines the injectable threshold value for each ingredient, and informs the display control portion of the threshold values. This is because the tolerable level of a given substance may vary dependent on with which ingedients it is coexistent. The display control portion 106 causes the threshold values to be displayed. The injectable threshold of each ingredient is represented by a dotted line in Fig. 10.

Let's assume that a patient designated as DB1021 is fed to the system. The control portion 101 look through the DB for DB1021, and fetches the data of DB1021 (for example, height and age), and delivers the data to the threshold determining portion 105. The threshold determining portion 105 determines following parameters for a given medicine: single dose, upper tolerable limit and lower effective limit for a daily dose, and alarming levels for upper and lower limits. The threshold determining portion 105 delivers those values to the control portion 105. The control portion 105 watches how the medicine is actually administered, and compares the actual dose with the thresholds. If the actual dose rises above or falls below those thresholds, the control portion causes the system to deliver an alarm. The alarm may occur as a sound or as a message on display 107.

The control portion 101, if it finds that the actual dose informed by the input portion 103 exceeds the injectable threshold, instructs the display control portion 106 to inform the user of the fact as well as to what extent the actual dose is over the threshold. The display portion 106 displays the actual dose in the display panel 21 for graph (as a histogram), and highlights the fraction exceeding the threshold.

When an intravenous dripping bottle must be exchanged, the user or physician instructs the speed of fluid supply to the system. The instruction is fed via the input portion 103. When the instruction is fed, the control portion 101 delivers the instruction to an exchange timing determination portion 104. The exchange timing determination portion 104 determines the timing of exchange based on the volume of the dripping fluid bottle and the instruction given by the user. The exchange timing determination portion 104 delivers the thus obtained timing to the display control portion 106. The display control portion 106 causes the exchange timing to be marked on a graphical displayed on the graphical display panel 21. The mark consists of a bottle symbol in this particular embodiment.

If the user feeds data via the input portion 103 to the display panel for treatment history of Fig. 10, the control portion 101 delivers the data to the display control portion 106. The display control portion causes the data to be graphical displayed.

The master file 1022 shown in Fig. 12 contains a table linking a medicine with a site to be applied. Therefore, the user can combine the trace records in the display panel 21 with the human figure shown in Fig. 23 based on the table of Fig. 10. For example, let's assume that the user chooses a certain site on the human body by feeding an input through the input portion 103. The control portion 101 looks through the table of master file 1022, and finds a medicine to be applied to the selected site, and delivers the data of the medicine to the display control portion 106. The display control portion 106 causes the medicine to be displayed highlighted. If the user feeds an input via the input portion 103 to choose a certain medicine listed on the display panel 21 for trace records, the control portion 101 looks through the table of master file 1022, and finds a site at which the medicine should be applied, and delivers the data of the site to the display control portion 106. The display control portion 106 causes the site 23 to be displayed highlighted. The highlighting includes enlargement, modified background, blinked letters, etc.

If the user wants to administer a number of medicines through the same route, and to check whether any adverse interaction may occur among those medicines, he/she firstly feeds the site 23 through which administration will occur via the input portion 103. On receipt of the input, the control portion 101 looks through the table of the master file 1022, and picks up combinations of medicines which may evoke adverse interaction when administered through that route, and delivers the result to the display control portion 106. The display control portion 106 causes the those hazardous combinations of medicines to be displayed.

The table of the master file 1022 is not limited to what is shown in Fig. 12, but may take any desired style. Namely, the table may take any style as long as medicines to be used and possible adverse combinations among them when administered through a certain chosen route can be readily recognized through the table. The chosen parameters and their values of the table represent an example, and are not limited to what is shown in the figure.

The feature of the display will be described with reference to Fig. 10. As described above, Fig. 10 shows the scheduled treatments in a sequential order along a time axis. In this particular embodiment, for a number of medicines, their scheduled administration times are indicated. On the top of Fig. 10 there is provided the same menu bar 18 as that of Fig. 4. Beneath the bar 18 there is provided a region 19 for indicating the patient characteristics.

Below the region there is provided a display panel 21 for indicating drug administrations and treatments (bolus injections and dripping injections) arranged on a time axis. In the vertical direction, there are provided a number of rows to be assigned to individual medicines. Thus, the user can see, for a number of medicines, when they should be administered.

In this particular embodiment, the rows are assigned to albumin, DOA, myoblock, heparin, lasix, cepharin, hedcylin, gastor, etc., and their sequential doses which are plotted on the ordinate are arranged on the time axis. Thus, the user can see at a glance for a given medicine when and at what dose it was given to the patient. The rightmost vertical line represents the current time. When a given medicine was administered at a scheduled time at a prescribed dose, that dosing is indicated by dots having a specified color, e.g., blue. On the contrary, if a certain administration is done at a dose exceeding the prescription, it is indicated by dots having another specified color, e.g., green together with the prescribed dose. The medicine may be exchanged for another, depending on the patient to be administered, and the row assigned to a medicine will be eradicated if it becomes unnecessary. Medicines to be administered are assigned to the upper rows downward in order. Thus, there are no vacant rows between any adjacent active rows.

If a medicine is injected, a check mark is given at a time when the injection occurred.

As described above, the system automatically determines, for a given bottle for intravenous dripping injection, the expected time of exchange based on the volume of the bottle and the speed of fluid supply instructed by the physician, and indicates it by placing a specified mark at a proper position on the time axis. From these data, nurses can adjust their visit to the patient, and can effectively avoid the failure of carelessly neglecting the exchange of an empty bottle. Because the system indicates the actually performed treatments along with the scheduled treatments, the user can readily grasp whether treatment is properly applied to a given patient.

Right to the display panel 21 there is provided a display panel 22 for displaying treatment history as in the display of Fig. 4. This display panel includes rows for indicating the instructions, the name of persons or physicians who gave the instructions, the name of persons who received the instructions, the time at which the instructions should be performed, the name of persons who practiced the instructions, etc. There are a number of columns for medicines to be used. In this particular embodiment, they are assigned to albumin, DOA, mioblock, heparin, lasix, cephalin, pentcillin, gaster, etc. Therefore, by watching the display panel 21 for treatment history, the user can see, for a number of medicines to be used, actually performed administrations along with the instructions regarding the administrations. Those data are also linked with the data given in the display panel 21, and thus, if an instruction is entered, a content of the instruction is graphical displayed in the display panel 22.

The display of Fig. 10 further shows a human body on the lower right corner. On the human body 23, the sites to which treatment should be applied are marked, and the sites are connected via lines with their names listed on the margins. In this particular embodiment, if the user chooses a site by clicking its name button, the medicines to be applied to that site and their related instructions are highlighted on the same display. On the contrary, if the user chooses a medicine on the display panel 21 by clicking its assigned row, the site to which the medicine should be applied is highlighted on the human body. Accordingly, medical practitioners can immediately tell, for a given medicine, at which site the medicine should be applied, or conversely for a given site on the human body, what medicine should be applied there. For example, medical practitioners can see at a glance which dripping medicinal fluids should be given through which routes for a given patient.

When it is required to apply the dripping injection of a medicine to a patient, generally the nurse determines through which route the dripping fluid should be given on the bedside. If the nurse decides to choose a certain route, and wants to check, prior to actual practice, whether any ingredients of the dripping fluid, if given through the candidate route, may have some adverse interaction with the medicines currently administered to the patient, it is often difficult for her to obtain necessary information at a timely manner. With this system, the nurse only needs to click her desired route on the human body. Then, she can check whether the dripping fluid contains any ingredients that may cause adverse interactions with the medicines currently given to the patient.

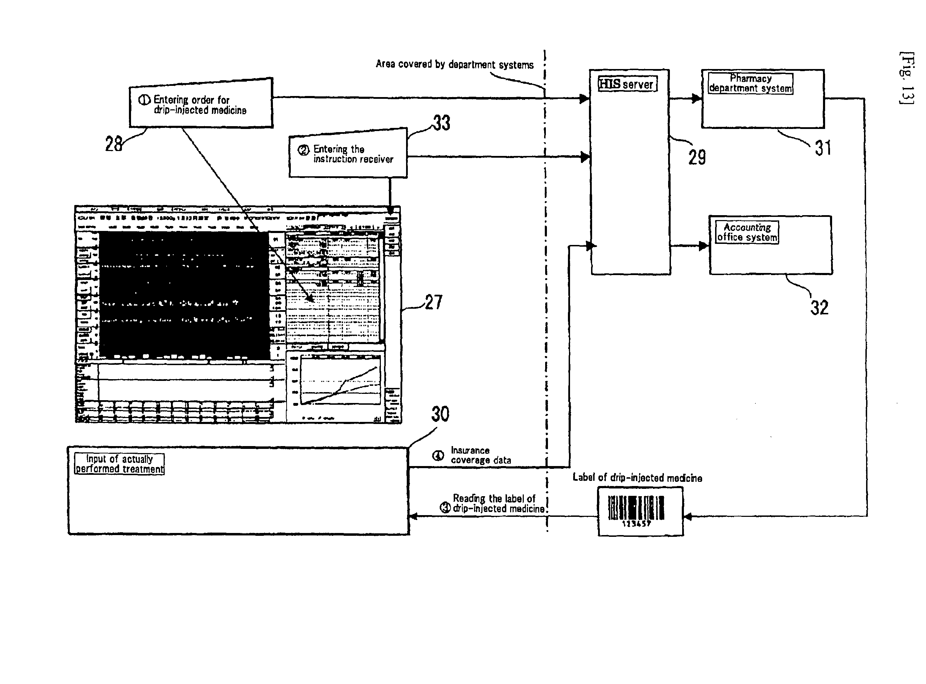

The system can store a database in which medicines are related with diseases and treatments. With the system having such a database, it is possible for the user to quickly know, for a given medicine, the name of medicines with which the medicine will have adverse interaction, by simply feeding the name of the medicine to the system. For example, the display of Fig. 13 shows medicines and their prescriptions necessary for a chosen medical treatment. If the user feeds the name of a medicine together with necessary instructions, he/she can obtain the name of medicines with which the studied medicine will have adverse interactions (row 24 for prohibited medicines). In the same manner, the user can know the name of disease (row 25 for prohibited medicines depending upon the name of diseases) to which the studied medicine may have adverse effects. The user can feed the upper tolerable limit and lower effective limit of a medicine for a given patient (row 26).

If the user feeds the upper and lower limits of a medicine on the display panel of Fig. 11, the system monitors the actual dosing of the medicine and, as soon as it finds that a certain dose is outside the specified range, it gives an alarm.

Next, the operations the user can effect via the display panel for treatment history of Fig. 10 will be described together with related display panels.

To gain access to the display panel for treatment history, the user may click the treatment history button on the menu bar 12 on the basic display of Fig. 4 (step S25). On the display panel for treatment history of Fig. 10, the user can choose any one from among the instruction mode, recording mode and review mode, by clicking an appropriate button on the upper right corner (step S26). If the user chooses the instruction mode, the user can feed his/her instruction to the system by clicking step S27 or S32 on the right of the display. At this time, the display panel 22 for treatment history includes rows for the instructions regarding dripping injection and bolus injection for a number of medicines.

The user or physician chooses a desired row by clicking it, and feeds a necessary instruction in that row. If the instruction includes administration of a drug which may adversely interact with a medicine currently given to the patient, the system warns the physician by giving an alarm (step S30). The warning may be given as a sound or a message on display. This warning may help the physician to avoid the blunder of carelessly instructing to give a hazardous drug to the patient. If the user chooses the recording mode, the system provides the user with the trace records of vital functions, or allows the user to review previously recorded data, although in this mode the user cannot feed any input to the system.

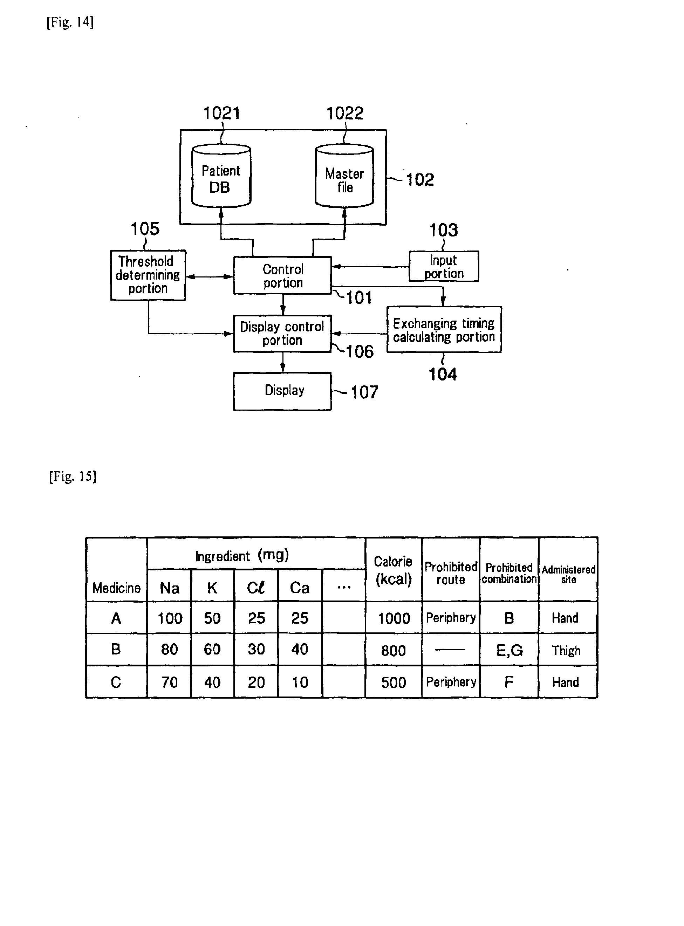

If the user chooses the recording mode as shown in Fig. 14, the system reads the bar code of the medicine nominated in the instruction mode (step S33). Then, the step hightlights the instruction related with the medicine (step S34). If the user or instructor checks the highlighted instruction and confirms that the instruction is rightly represented, he/she feeds his/her affirmative answer to the system (step S35). When the system receives the affirmative answer from the user, it urges the instruction to be put into practice (step S37). Namely, the user feeds the dose and clicks the end button (step S38). Then, the system reads the bar code of the next drug (step S39). If the user chooses the review mode, the user can have display panels for a graphic display of instructions, detailed instructions, and a figure of the human body at the same time (steps S40 and S41). If the user clicks the history button, he/she can view the history of a patient at a desired time span (steps S42 and S43).

If the user wants to change a previously given instruction, he/she chooses the instruction mode, and feeds a new instruction to be exchanged for the previous instruction to the system (steps S44 to S48). For the new instruction, if it is outside the specified tolerable range, the system warns the user by giving an alarm (step S46).

If the user wants to change a medicine or a route for dripping injection previously given, he/she may operate on the system similarly to above (steps S49 to S53). However, if the new medicine or new route for dripping injection has the risk of causing an adverse interaction, the system will warn the user by giving an alarm (step S51).

The system also reads the bar code of a treatment actually performed and stores it (step S54). These data are displayed on the display panel 13 for treatment history on the right side of the display of Fig. 4, and on the display panel 21 for treatment history of Fig. 10.

The flow of information through the present system will be described with reference to Fig. 15, based on an example where a medicine is administered via bolus injection or dripping injection to a patient under the instruction from a physician.

As described above, the user or physician feeds an instruction via the display panel 13 for treatment history of the basic display 27, by using an input feeding device such as a mouse. In this example, the user feeds an instruction regarding the dripping injection of a medicine. This instruction regarding the dripping injection of a medicine is transmitted to the server 29 of a hospital beyond the peripheral systems of individual departments. With regard to the dripping injection of a medicine, the user or physician feeds the name of vehicle, the name of instruction giver, the name of instruction receiver, the name of injector, the time of injection, the method of injection (dose, speed and route), etc. The input feeding means includes, in addition to a keyboard, a scanner. For example, the name of a medicine can be fed to the system by allowing a scanner to read the bar code attached to the bottle of the medicine. This mode of input feeding can also be applied to the name of instruction giver, name of instruction receiver, details of treatment, if the necessary information is given as bar codes on the name plates, medical records, etc.

When the user wants to make dripping injection of a medicine, he/she takes the following procedures.
1) The user allows a scanner to read the bar code of a bottle containing a vehicle to inform the system of the vehicle. The system checks that the vehicle is the same as previously given in the corresponding instruction.
2) The system informs the user of the affirmative answer. The user determines a rout through which dripping injection will be made. The user informs the system of the route he/she has chosen, by pointing to an appropriate site on the figure of human body.
   The system automatically checks whether any ingredients of the vehicle and medicine might have adverse interaction with medicines currently given to the patient, if the medicine in the vehicle were injected through the chosen route into the patient's body.
3) If the system finds the treatment in question safe, it presents an affirmative message close to an image of bottle on the right side of the display which represents the treatment in question.
4) As soon as the user starts the dripping injection of a medicine, he/she clicks "Start." The bottle image on display changes its color. If the user wants to discontinue the injection midway, he/she clicks the cancel button.
5) The user feeds to the system the injection volume of a medicine at one hour intervals.

If the system compares the volume in question with the volume given in the corresponding instruction, and finds the two are the same, it sends an affirmative answer to the user. While the system continues to send affirmative answers, the monitored time splits have a specified color (blue). When the user receives a negative answer from the system, he/she checks the comparison result, corrects the injection of a medicine according to the comparison result, and feeds new corrected data to the system. While injection based on the corrected data proceeds, the corresponding time splits have another specified color (e.g., green).

If the user wants to know the injected amount of a medicine at a certain time in the future, he/she clicks the corresponding time on the time axis in the row for the medicine. Then, the system automatically calculates the expected injection amount at that time, and informs the user of that amount and whether the amount corresponds with the instruction or not.

If the user exchanges a bottle for dripping injection for a new one, the system checks whether fluid in the new bottle contains any ingredients possibly hazardous to the patient as described above, using the steps different from those described in 2).

When practicing a treatment lasting briefly such as bolus injection of a medicine, the user allows a scanner to read the bar code of the medicine. The system compares the currently fed medicine with the medicine notified in the instruction, and if it finds the two are the same, it then gives a message to urge the user to review the original instruction below the injection mark corresponding to the treatment on the display panel for trace records of Fig. 10.

When the system compares the actually performed treatment with the instruction and finds the two are the same, it gives an affirmative answer. Then, the injection mark changes its color (e.g., blue). The system determines the end of the treatment and closes the input 30 for the treatment.

If the system finds the actually performed treatment is different from the instruction, the system informs the user of the comparison result. The user then checks the comparison result, corrects the injection of a medicine according to the comparison result, and feeds new corrected data to the system. Then, the injection mark changes its color (e.g., green).

The data fed by the user is stored in a terminal of the user's department, and then transmitted to an HIS server of the hospital which is responsible for the management of data handled by all the departments of the hospital. Part of data stored in the HIS server is transmitted to a system 31 of the pharmacy department where various kinds of medical data are distributed to personnel specialized in individual medical fields.

When practicing a treatment such as the dripping injection of a medicine, the user or practitioner practices the treatment according to the instruction while monitoring the display, allows a scanner to read the bar code attached to a bottle containing the medicine, and feeds the data of the actually performed treatment. All those are transmitted to the HIS server. When the treatment lasts long as with the dripping injection of a medicine, the treatment data is collected at regular intervals, and transmitted to the HIS.

HIS compares the actually performed treatment with the corresponding instruction, and transmits the comparison result to the user's terminal. The user's terminal also calculates the cost required for the treatment including the fraction covered by insurance, and transmits the calculation result to the HIS server. HIS collects these cost and insurance data from all the departments, and transmits the collected data to a system 32 of the accounting office.

The user or instruction receiver can also feed input 33 manually or automatically. In short, all the data regarding the instructions of treatment, and treatments actually performed by every medical practitioner are managed by this system in a systematic way.

As described above, according to the present invention, it is possible to provide a system which can minimize the risk of making medical blunders as a result of erroneous input/instructions, missed input/instructions or duplicate input/instructions.

It is further possible to provide a system which can promote the activity of the staff of ICU with different backgrounds by helping them to smoothly deliver, receive and achieve treatment instructions.

It is still further possible to provide a patient information management system capable of safely storing patient information in a style applicable to an electronic medical record system.

This invention is based on the Japanese Patent Application No. 2001-205818 filed on July 6, 2001. This is incorporated in its entirety herein by reference.

### Industrial Applicability

This invention will be greatly beneficial for the medical practitioners working in the wards of hospital where seriously sick patients are cared such as ICU and NICU, in constructing a therapeutic plan or care plan, or in storing data to serve as a material of electronic medical records.

## Claims

1. An apparatus for managing patient information comprising:
at least one condition detecting means for detecting patient conditions;
detected value displaying means for outputting a detected value obtained in said condition detecting means to display the detected value on a display screen;
corrected value inputting means for inputting a corrected value of the detected value in the display screen displayed by said detected value displaying means corresponding to said condition detecting means; and
corrected value displaying means for displaying said corrected value inputted by said corrected value inputting means on said display screen that said detected value is displayed.

2. The apparatus according to claim 1, further comprising display stopping means for stopping displaying the detected value with regard to a predetermined detected value.

3. The apparatus according to claim 1, wherein said condition detecting means includes at least two means selected from the group consisting of:
heartbeat detecting means for detecting a heartbeat of said patient;
arterial pressure detecting means for detecting an arterial pressure of said patient;
saturated-oxygen-in-blood detecting means for detecting saturated-oxygen in blood of said patient;
center venous pressure detecting means for detecting a center venous pressure of said patient;
left atrium pressure detecting means for detecting a left atrium pressure of said patient;
pulmonary artery pressure detecting means for detecting a pulmonary artery pressure of said patient;
brain pressure detecting means for detecting a brain pressure of said patient; and
respiratory rate measuring means for measuring a respiratory rate of said patient.

4. An apparatus for managing patient information comprising:
provisional treatment displaying means for display the relationship between a content of a provisional treatment to a patient and the time;
completed treatment displaying means for displaying a content of a completed treatment to said patient with the time; and
decision result displaying means for deciding whether or not said content of provisional treatment matches with said content of completed treatment and for displaying a decision result.

5. The apparatus according to claim 4, wherein the apparatus has a first display screen displaying said provisional treatment, said completed treatment and said decision result and a second display screen displaying a relationship between a human body chart and a body part to be treated, and said first and second display screen can be seen at the same time.

6. The apparatus according to claim 5, further comprising link displaying means for displaying said provisional treatment, said completed treatment and said decision result in the first displaying screen, and said body part in the second displaying screen linked with each other.

7. The apparatus according to claim 4, wherein said provisional treatment displaying means displays a treatment content graphically.

8. The apparatus according to claim 4, wherein said completed treatment displaying means displays a treatment content graphically.

9. An apparatus for managing patient information comprising:
at least one condition detecting means for detecting patient conditions;
detected value displaying means for outputting a detected value obtained in said condition detecting means to display the detected value on a display screen;
corrected value inputting means for inputting a corrected value of the detected value in the display screen displayed by said detected value displaying means corresponding to said condition detecting means;
corrected value displaying means for displaying said corrected value inputted by said corrected value inputting means on said display screen that said detected value is displayed;
provisional treatment displaying means for display the relationship between a content of a provisional treatment to a patient and the time;
completed treatment displaying means for displaying a content of a completed treatment to said patient with the time;
decision result displaying means for deciding whether or not said content of provisional treatment matches with said content of completed treatment and for displaying a decision result; and
screen switching means for switching a display screen displaying said detected value and/or said corrected value, and a display screen displaying said provisional treatment, said completed treatment and said decision result.

10. A method for managing patient information comprising the steps of:
detecting at least one patient condition by condition detecting means and a monitor connected to said condition detecting means in order to grasp a patient condition and outputting a detected result of said condition detecting means on a screen;
correcting a monitor output on the basis of said monitor output displayed and displaying a corrected value on monitor screen with said monitor output;
display the relationship between a content of a provisional treatment to a patient and the time on a display screen;
displaying a content of a completed treatment to said patient with the time;
deciding whether or not said content of provisional treatment matches with said content of completed treatment and for displaying a decision result; and
switching a display screen displaying said detected value and/or said corrected value, and a display screen displaying said provisional treatment, said completed treatment and said decision result and display either display screen.

11. A program to execute a method according to claim 10.
